# EUROPEAN PATENT SPECIFICATION

(11) **EP 3 184 681 B1**
(45) Date of publication and mention of the grant of the patent: **14.08.2019**
(21) Application number: 15834441.6
(22) Date of filing: 26.03.2015
(51) Int. Cl.: D04H 1/4391, A61F 13/15, A61F 13/53, D04H 1/435

(54) **A WATER ABSORPTION SHEET FOR SANITARY NAPKINS COMPRISING A NON-WOVEN FABRIC FOR SANITARY MATERIALS**
WASSERABSORBIERENDE LAGE FÜR DAMENBINDE ENTHALTEND EINEN VLIESSTOFF FÜR SANITÄRMATERIALIEN
FEUILLE ABSORBANTE POUR SERVIETTE HYGIÉNIQUE COMPRENANT UN TISSU NON-TISSÉ POUR MATÉRIAUX SANITAIRES

(30) Priority: 20.08.2014 JP 2014167409
(43) Date of publication of application: 28.06.2017
(73) Proprietor: Toray Industries, Inc., Tokyo 103-8666 (JP)
(72) Inventor: KASAHARA, Teruhiko, Ehime 791-3193 (JP); TANINOMIYA, Masahiro, Ehime 791-3193 (JP); KINOSHITA, Yusuke, Ehime 791-3193 (JP)
(74) Representative: Hoefer & Partner Patentanwälte mbB
(86) International application number: PCT/JP2015/059371
(87) International publication number: WO 2016/027493

(56) References cited:
- EP-A1- 2 881 505
- EP-A1- 2 963 168
- EP-A1- 2 980 288
- WO-A1-2014/132690
- JP-A- H0 424 214
- JP-A- H0 424 214
- JP-A- H02 234 915
- JP-A- H02 251 649
- JP-A- H04 136 217
- JP-A- H10 158 953
- JP-A- H11 508 970
- JP-A- 2005 350 777
- JP-A- 2005 350 777
- JP-A- 2015 067 916
- US-A1- 2013 133 980

## Description

### Field

The present invention relates to a water absorption sheet for sanitary napkins comprising a nonwoven fabric for sanitary materials that is high in water absorbability, high in liquid holding capacity for water, chemicals, etc., and high in bulkiness, flexibility, and anti-see-through property. Furthermore, disclosed are sanitary material products using the nonwoven fabric for sanitary materials.

### Background Art

Having various good characteristics including high strength, high heat resistance, high chemical resistance, and good wash and wear properties, synthetic fibers such as polyester fiber and polyamide fiber have conventionally been used in a wide variety of products including clothing and industrial materials. In recent years, polyester fiber, among other synthetic fibers, is playing an increasingly important role in the field of nonwoven fabrics, particularly in the field of sanitary materials such as baby diapers, diaper liners, and sanitary articles; living materials such as counter cloth and wet tissues for the restaurant industry; non-sanitary materials such as throating bags for sinks in kitchen utensils; medical materials such as base fabrics and fixing sheets for poultices, hospital surgical gowns, and masks; and industrial materials such as automotive interior materials and filters. As a surface material for sanitary goods such as disposable diapers, sanitary napkins, incontinence pads, and wound dressing, in particular, polyester fiber has come in wider use with the aim of cost reduction and increased bulkiness. Surface materials in disposable diapers and sanitary napkins are required to have improved water absorbency, light weight, strength, and dry feeling, as well as excellent texture and bulkiness.

To meet such requirements, some surface materials have been proposed including nonwoven fabric that includes a main fiber and a binder component in which the monofilament cross-section of the main fiber has a flatness of 2.0 or less and has two or more openings with an aperture angle of less than 120 degrees (see Patent document 1). However, although a cross-sectional shape as specified in this proposal can ensure a high water absorbability and bulkiness, a fiber cross section with a flatness of 2.0 or less leads to poor bristle falling properties, failing to provide nonwoven fabrics having good texture with soft feeling to the skin.

In addition, there is a study that proposes a surface material that contains an olefin-type composite fiber with a multilobar fiber cross section and that serves as surface material for lightweight, bulky paper diapers and sanitary articles (see Patent document 2). The material proposed here, however, is still poor in water absorbability and nonwoven fabric strength although it is high in bulkiness and texture from the viewpoint of a cross-sectional shape and polyolefin based fiber. Furthermore, the body fluid absorbed in the water absorption layer can be seen through the material, causing problems such as unfavorable visual features and discomfort.

There is another study that proposes a disposable sanitary material that is characterized by having a back sheet of nonwoven fabric containing a hydrophobic synthetic fiber with an irregular cross-sectional shape (see Patent document 3). Materials with such a proposed cross-sectional shape, however, are still insufficient in such features as anti-see-through property and water absorbability of the water absorption layer although having excellent flexibility for use as a back sheet of sanitary material.

### Prior Art Documents

### Patent Documents

Patent document 1: Japanese Unexamined Patent Publication (Kokai) No. 2012-197546
Patent document 2: Japanese Unexamined Patent Publication (Kokai) No. 2000-345456
Patent document 3: Japanese Unexamined Patent Publication (Kokai) No. 2003-319970

EP 2 963 168 A1, which is state of the art pursuant to Art. 54(3) EPC, discloses a nonwoven fabric comprising 20 to 80 % by weight of a polyester fiber having an oblate multilobal cross-section and 20 to 80 % by weight of a cellulose fiber, and the polyester fiber has an oblate multilobal cross-section with an oblate cross-section having at least 6 lobes on its periphery.

EP 2 980 288 A1, which is state of the art pursuant to Art. 54(3) EPC, relates to a mixed spun yarn which comprises 20 to 80 mass% of a polyester-based fiber having a flat multilobe section and 20 to 80 mass% of a cellulose-based fiber.

EP 2 881 505 A1, which is state of the art pursuant to Art. 54(3) EPC, concerns a fabric subjected to calender processing on one or both surfaces, comprising a polyamide fiber as warp and/or woof having, after calender processing, a single filament fineness of 0.5 to 2.5 dtex and a total fiber fineness of 5 to 50 dtex, the single filament having a cross-sectional shape that is flat multifoliar with 6 to 10 lobe parts and has a flat ratio (W) (α/β) of 1.5 to 3.0, the fabric having a cover factor of 1200 to 2500.

JP 2005 350777 A discloses a polyester fiber consisting of a single fiber having ≤ 2.5 dtex single fiber fineness and a flat multilobed shape of 6-10 lobes of the single fiber cross-sectional shape.

JP H04 24214 A discloses a modified polyester containing a metal sulfonate group-containing isophthalic acid in an amount of 0.7-2.4 mol% based on dicarboxylic acid components and a polyalkylene glycol of 90-6000 molecular weight in an amount of 0.2-10 wt.-% based on the polyester is spun to obtain a fiber.

### Summary of the Invention

### Problems to be Solved by the Invention

Thus, an object of the present invention is to provide a water absorption sheet for sanitary napkins comprising a nonwoven fabric for sanitary materials having high water absorbability and high diffusing capacity for liquids such as water and chemicals as well as high bulkiness, flexibility, and anti-see-through property, and also provide sanitary material products using this nonwoven fabric for sanitary materials.

### Means of Solving the Problems

Aiming to solve these problems, the present inventors found that a water absorption sheet for sanitary napkins comprising a nonwoven fabric for sanitary materials that is high in water absorbability, bulkiness, and flexibility can be obtained by using a polyester based fiber having a specific fiber cross-sectional shape, and arrived at the present invention after further intensive studies.

The water absorption sheet for sanitary napkins comprising a nonwoven fabric for sanitary materials according to the present invention is nonwoven fabric including a polyester based fiber with a flattened multilobar cross section in which 6 or more convex portions exist along the circumference of the fiber cross section, the surface of the nonwoven fabric for sanitary materials being covered with olefin based nonwoven fabric, and the polyester based fiber having a flattened multilobar cross section in the nonwoven fabric for sanitary materials simultaneously meet the requirements (1) to (3) described below.
- flatness (A/B) = 2.0 to 3.0 (1)
- degree of irregularity (C/D) = 1.0 to 5.0 (2)
- convex ratio (E/B) = 0.6 to 0.9 (3)
where
A: A denotes the maximum length of the fiber cross section,
B: B denotes the maximum width of the fiber cross section,
C: C denotes the length of the line connecting the apexes of the adjacent convex portions in the largest irregular shape part of the fiber cross section,
D: D denotes the length of the line that extends perpendicularly from the line C connecting two convex portions to the bottom of the concave portion, and
E: E denotes the length of the longest line except for the maximum width B of the cross section.

According to a preferred embodiment of the water absorption sheet for sanitary napkins comprising a nonwoven fabric for sanitary materials of the present invention, the polyester based fiber having a flattened multilobar cross section has a single filament fineness of 2.0 dtex or less.

According to a preferred embodiment of the water absorption sheet for sanitary napkins comprising a nonwoven fabric for sanitary materials of the present invention, the polyester based fiber having a flattened multilobar cross section has a fiber length in the range of 3 mm to 64 mm.

According to a preferred embodiment of the water absorption sheet for sanitary napkins comprising a nonwoven fabric for sanitary materials of the present invention, the polyester based fiber having a flattened multilobar cross section is nonwoven fabric in which fiber joining is achieved with heat-weldable fiber.

The sanitary material products which are not part of the present invention are sanitary material products including the nonwoven fabric for sanitary materials.

According to a preferred variant of the sanitary material products which are not part of the present invention, the nonwoven fabric for sanitary materials has a surface covered with olefin based nonwoven fabric.

According to the present invention, a water absorption sheet material for sanitary napkins can be produced using the nonwoven fabric for sanitary materials.

### Advantageous Effect of the Invention

The present invention can provide water absorption sheet for sanitary napkins comprising a nonwoven fabric for sanitary materials having high water absorbability and high diffusing capacity for liquids such as water and chemicals as well as high bulkiness, flexibility, and anti-see-through property. The water absorption sheet for sanitary napkins comprising a nonwoven fabric for sanitary materials according to the present invention is particularly high in flexibility and water absorbing and diffusing capacity and also has other various good features such as anti-see-through property.

According to the present invention, furthermore, water absorption sheets for sanitary napkins can be produced using the nonwoven fabric for sanitary materials.

### Brief Description of the Drawings

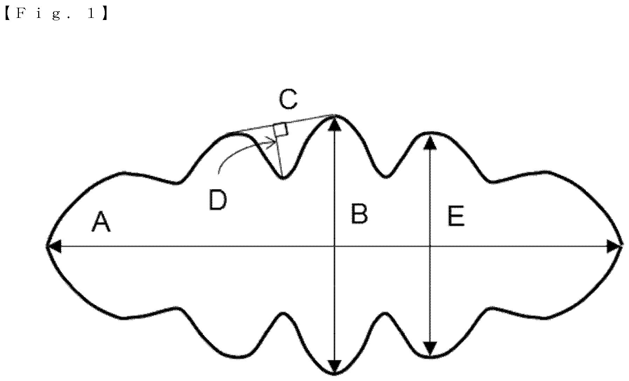

[Fig. 1] Fig. 1 is a cross-sectional diagram for illustrating the cross-sectional shape of the polyester fiber with a multilobar flattened cross section in nonwoven fabric for sanitary materials used for a water absorption sheet for sanitary napkins according to the present invention that has a plurality of (eight) convex portions along the circumference of the fiber cross section.

### Description of Preferred Embodiments

Described in detail below is the water absorption sheet for sanitary napkins comprising a nonwoven fabric for sanitary materials and the sanitary material products which are not part of the present invention.

The water absorption sheet for sanitary napkins comprising a nonwoven fabric for sanitary materials according to the present invention is nonwoven fabric including a polyester based fiber with a flattened multilobar cross section in which 6 or more convex portions exist along the circumference of the fiber cross section, the surface of the nonwoven fabric for sanitary materials being covered with olefin based non woven fabric, and the polyester based fiber having a flattened multilobar cross section in the nonwoven fabric for sanitary materials simultaneously meet the requirements (1) to (3) described below for the flatness, the degree of irregularity, and the convex ratio.
- flatness (A/B) = 2.0 to 3.0 (1)
- degree of irregularity (C/D) = 1.0 to 5.0 (2)
- convex ratio (E/B) = 0.6 to 0.9 (3)
where
A: A denotes the maximum length of the fiber cross section,
B: B denotes the maximum width of the fiber cross section,
C: C denotes the length of the line connecting the apexes of the adjacent convex portions in the largest irregular shape part of the fiber cross section,
D: D denotes the length of the line that extends perpendicularly from the line C connecting two convex portions to the bottom of the concave portion, and
E: E denotes the length of the longest line except for the maximum width B of the cross section.

As described later, it is important for a polyester based fiber having a flattened multilobar cross section used for the present invention to have a cross section in which 6 or more, preferably 8 or more, convex portions exist along the circumference. As for the shape of each concave or convex portion on the circumference of the cross section, furthermore, it is preferably a curved shape from the viewpoint of feel to the skin.

The polyester as referred to here to form the polyester based fiber for the present invention may be a polyester polymer produced through condensation reaction between terephthalic acid and ethylene glycol or butylene glycol, a condensation product between sebacic acid, adipic acid, trimellitic acid, isophthalic acid, parahydroxybenzoic acid, etc., and ethylene glycol or butylene glycol, or any of other various polyesters.

The polyester based fiber having a flattened multilobar cross section used for the present invention is a polyester based fiber that has a flattened cross section containing 6 or more convex portions. If there are only less than 6 convex portions on the circumference of the cross section, a sufficiently large void will not be formed between adjacent fibers, resulting in an insufficient water absorbability or liquid holding capacity. In addition, a flattened cross-sectional shape makes it possible to form an interfiber void, leading to high bulkiness. Furthermore, the nonwoven fabric is low in the degree of bristle falling per monofilament and accordingly able to have a soft texture. For the present invention, the term "multilobar" means that the circumference of the fiber cross section has many convex portions.

Fig. 1 gives an example of the single filament cross section of the polyester based fiber having a flattened multilobar cross section used for the water absorption sheet for sanitary napkins of present invention. Fig. 1 illustrates a cross-sectional shape of the polyester fiber with a multilobar flattened cross section in nonwoven fabric for sanitary materials according to the present invention that has a plurality of (eight) convex portions along the circumference of the fiber cross section.

As described above, the water absorption sheet for sanitary napkins of the present invention uses a polyester based fiber that has a flattened cross section containing 6 or more convex portions, but preferably 8 or more, more preferably 10 or more, convex portions should be contained. In addition, the upper limit of the number of such convex portions is preferably 12. Furthermore, each convex portion preferably has a curved shape from the viewpoint of feel to the skin.

The polyester based fiber having a flattened multilobar cross section used for the water absorption sheet for sanitary napkins of the present invention is formed of polyester based fiber in which the flattened multilobar cross-sectional shape of the monofilament cross section simultaneously satisfies the following requirements: a flatness as represented by Equation (1) given below, a degree of irregularity as represented by Equation (2) given below, and a convex ratio as represented by Equation (3) given below:
- flatness (A/B) = 2.0 to 3.0 (1)
- degree of irregularity (C/D) = 1.0 to 5.0 (2)
- convex ratio (E/B) = 0.6 to 0.9 (3)

Here, as shown in Fig. 1, the above parameter A denotes the length of the longest line segment in the flattened multilobar cross section. The above parameter B denotes the length of the line segment that is perpendicular to the line segment A and represents the maximum width between two convex portions. The above parameter C denotes the length of the line segment that connects the apexes of the adjacent convex portions contained in the largest convex-concave region in the flattened multilobar shape. The above parameter D denotes the length of the line segment that extends perpendicularly from the line connecting two convex portions to the bottom of the concave portion. The above parameter E, furthermore, denotes the length of the second largest width next to the largest width B in the cross section.

The flatness, degree of irregularity, and convex ratio referred to herein are calculated from measured sizes that are determined from photographs with a magnification of 400 times of a cross section of prepared fibers using a biological microscope and enlarging the cross-sectional photographs by 300% using a copying machine.

Specifically, according to a preferred embodiment of the nonwoven fabric for a water absorption sheet for sanitary napkins comprising sanitary materials of the present invention, the polyester based fiber having a flattened multilobar cross section used for the nonwoven fabric for sanitary materials includes polyester based fiber that simultaneously meet Equation (1) given above for the flatness, Equation (2) given above for the degree of irregularity, and Equation (3) given above where: A denotes the largest length across the cross section; B denotes the largest width; C denotes the length of the line that connects the apexes of the adjacent convex portions contained in the largest convex-concave region; D denotes the length of the perpendicular line from the line connecting the apexes of the adjacent convex portions to the bottom of the concave portion; and E denotes the maximum length in the cross section except for the largest width B.

If the flatness (A/B) is less than 2.0, the fiber will have poor bristle falling properties and fail to have a soft texture. On the other hand, if the flatness (A/B) exceeds 3.0, the fiber will be low in bending strength, tend to wear out easily, and tend to be poor in bulkiness. In addition, the yarn-making performance and the degree of irregularity also tend to decline. The flatness (A/B) is preferably 2.0 to 2.7 and more preferably 2.0 to 2.5. For the present invention, the flatness influences the feel during the use of the sanitary material products, and a larger flatness ensures good bristle falling properties (fibers bend largely), leading to a soft feel when touched by the skin (feel to the skin).

The degree of irregularity (C/D) represents the size of the concave portion between two convex portions in the flattened multilobar shape. A larger value means a smaller concave portion while a smaller value means a larger concave portion. As the degree of irregularity (C/D) increases, the depth of the concave portion decreases and the size of the voids formed between fibers also decreases, possibly leading to a decrease in both water absorbability and water holding capacity and also to a decrease in the diffusion effect attributed to the capillary action during water absorption. Thus, the degree of irregularity (C/D) should be 5.0 or less. If the degree of irregularity (C/D) is too small, on the other hand, the fiber tends to be folded at a concave portion of the cross section, possibly failing to maintain a flattened shape. Furthermore, the fiber will be damaged easily by abrasion, possibly causing damage to the skin when undergoing friction with the skin. Accordingly, the degree of irregularity (C/D) should be 1.0 or more. From the above description, the degree of irregularity (C/D) should be in the range of 1.0 to 5.0. Furthermore, the degree of irregularity (C/D) should more preferably be in the range of 2.0 to 4.0 from the viewpoint of water absorbability and water holding capacity.

For the present invention, the degree of irregularity influences the water absorbability of the water absorption sheet for sanitary napkins comprising a sanitary materials and their feel to the skin after absorbing water. As this value increases excessively, the water absorbability and diffusing capacity tend to deteriorate, possibly making the liquid to collect at a particular point, which can cause deterioration in the dry texture of the product surface and discomfort to the skin.

In addition, the convex ratio (E/B) serves as an indicator of the degree of deformation of the nearly elliptic shape that is formed by drawing a line that connects the apexes of the convex portions associated with the maximum width of B, the width of E, and the maximum length of A. When the convex ratio is too small, the depth of the concave portion will be so small that the cross section will become excessively closer to a flattened cruciform shape. Accordingly, the interfiber voidage will become too high and the liquid diffusing capacity due to capillary action during water absorption will decrease. Furthermore, the fiber will deteriorate in bristle falling properties, leading to a decline in the feel to the skin and softness. In addition, the fiber will suffer from increased irregular reflection of light, leading to a large deterioration in the anti-see-through effect. Thus, the convex ratio should be 0.6 or more.

If the convex ratio is too large, on the other hand, the convex and concave portions of fiber may fit to each other, and the voidage may decrease due to an increased number of completely blocked concave portions, leading to a decrease in the diffusing capacity attributed to capillary action during water absorption. Furthermore, the blocking of concave portions due to such fitting causes a decrease in bulkiness, leading to the loss of bulging texture. Thus, the convex ratio (E/B) should be 0.9 or less. From the requirements described above, the convex ratio (E/B) should be in the range of 0.6 to 0.9. Furthermore, the convex ratio (E/B) is preferably 0.6 to 0.8 and more preferably 0.7 to 0.8 from the viewpoint of the balance.

For the present invention, the convex ratio is related with the feel to the skin (texture), water absorption, diffusing capacity, and bulkiness of the sanitary material products. As the convex ratio becomes too high, voids will disappear due to interfiber fitting, leading to a decline in water absorbability and bulkiness, whereas as the convex ratio becomes too small, the shape will become cruciform, possibly leading to a decrease in softness.

For the polyester based fiber having a flattened multilobar cross section used for the water absorption sheet for sanitary napkins of the present invention, the single filament fineness is preferably 2.0 dtex or less. The single filament fineness is more preferably 1.0 to 2.0 dtex and still more preferably 1.2 to 1.8 dtex. If the single filament fineness is more than 2 dtex, the rigidity characteristic of polyester fiber increases significantly and the stimulation associated with the feel to the skin also increases, possibly leading to a decline in soft texture. In addition, since voids formed among filaments will become too large, wet wipers produced from the fiber will be so low in liquid holding capability that the liquid can leak through the nonwoven fabric during use, and wet-back will occur easily through water absorption layers in surface material in diapers. If the single filament fineness is less than 1.0 dtex, on the other hand, the processability in a carding process will tend to be low, leading to a decrease in productivity.

For the polyester based fiber having a flattened multilobar cross section, furthermore, the fiber length is preferably 30 to 64 mm from the viewpoint of slipping-off of fibers from the nonwoven fabric for sanitary materials. The fiber length is more preferably 35 to 51 mm.

In a typical production method for the polyester based fiber having a flattened multilobar cross section used for the present invention, a spinneret containing special irregular-shaped holes for flattened multilobar cross section formation is used and polyethylene terephthalate with a molten polymer density of 1.10 to 1.20 g/cc is spun under the conditions of a spinning temperature of 280 to 300°C, a discharge rate of 300 to 400 g/min, a cooling air speed of 50 to 100 m/min, a cooling temperature of 10°C to 30°C, and a take-up speed of 1,100 to 1,300 m/min to produce an undrawn yarn. Subsequently, the resulting undrawn yarn is stretched 3.0 to 3.5 times and crimped under the conditions of 12 to 16 crimps/25 mm and a crimping degree of 12% to 18%, and the fiber surface is provided with 0.2 to 0.4 mass% of a hydrophilizing agent, followed by drying at a temperature of 100°C to 120°C. After drying, it is cut to 30 to 64 mm to provide polyester fiber with a flattened multilobar cross section as described in Fig. 1.

Next, according to a preferred embodiment of the nonwoven fabric for sanitary materials of the present invention, the polyester based fiber having a flattened multilobar cross section is joined using heat-weldable fiber to produce nonwoven fabric, which serves as material for a water absorption sheet in sanitary napkins covered with a top sheet of olefin based nonwoven fabric. Formed of the polyester based fiber having a flattened multilobar cross section, such a water absorption sheet material has high water absorbability, high flexibility, and good anti-see-through property. The heat-weldable fiber to be used for this water absorption sheet material may be a single-component type heat-weldable fiber, but it is preferable use a composite type heat-weldable fiber, such as of side-by-side type or sheath-core type, that contains two or more resin components including ones that do not undergo fusion bonding when heat-treated, because the portions left unbonded can maintain fiber strength. For the present invention, the content of such heat-weldable fiber is preferably 5 to 20 mass% relative to the mass of the nonwoven fabric.

The resin components that can be used as material for the heat-weldable fiber include combinations of 6 nylon and polyethylene, polypropylene and polyethylene, polypropylene and an ethylene-vinyl acetate copolymer, polyester and polypropylene, polyester and polyethylene, 6 nylon and 66 nylon, and high density polyester and low density polyester.

The heat-weldable fiber as referred to herein preferably has a melting point of 110°C to 160°C.

Furthermore, the water absorption sheet for sanitary napkins comprising a nonwoven fabric for sanitary materials according to the present invention may also contain cellulose based fiber in addition to the polyester fiber with a flattened multilobar cross section. The above cellulose based fiber is at least one selected from the cellulose based fiber group consisting of natural fibers such as hemp, cotton, and silk, regenerated fibers such as viscose rayon, cupra, and solvent spun cellulose, and semisynthetic fibers such as acetate. Of these, regenerated fibers such as viscose rayon and solvent spun cellulose are preferred from the viewpoint of handleability and versatility. If cellulose based fiber is used for the present invention, its content is preferably 5 to 20 mass% relative to the mass of the nonwoven fabric.

Described below is the production method for the nonwoven fabric for sanitary materials which are used in the water absorption sheet for sanitary napkins according to the present invention.

The above polyester based fiber having a flattened multilobar cross section and the heat-weldable fiber, cellulose based fiber, etc., are subjected to preliminary opening and mixing by using an opener and carded to produce a fiber web. The fiber web produced by carding is sent to a spunlace process by a feed lattice, entangled by a high pressure water stream, and processed into a sheet. The nonwoven fabric resulting from the spunlace processing is sent by a conveyor to a drying process. In the case where a heat-weldable fiber as described above is used, the air-through method is carried out for simultaneous drying and heat treatment at a temperature where only the heat-weldable fiber is melted, thus proving nonwoven fabric.

The nonwoven fabric produced above has high water absorbability and high diffusing capacity together with excellent bulkiness and soft texture and accordingly can serve very effectively as nonwoven fabric for sanitary materials such as water absorbable articles as intended by the present invention.

The nonwoven fabric thus obtained preferably has a targeted of 50 g/m² or less, more preferably 20 to 50 g/m², and particularly preferably 30 to 50 g/m². A targeted more than 50 g/m² can lead to deterioration in lightweight properties. A targeted less than 20 g/m², on the other hand, can lead to difficulty in ensuring stable formation of a carded web. Furthermore, the nonwoven fabric preferably has a thickness in the range of 2 to 10 mm (more preferably 2 to 5 mm) in order to ensure water absorbability, anti-see-through property, and wet-back prevention simultaneously.

Specifically, sheet materials for absorbable articles manufactured from nonwoven fabric for sanitary materials as produced above have high water absorbability and high diffusing capacity for liquids such as water and chemicals as well as excellent bulkiness, flexibility, and anti-see-through property and accordingly can serve very effectively as nonwoven fabric for sanitary materials to be used for sanitary napkins and paper diapers, which will come into contact with the skin. In addition to the above uses, they can also be applied to other sanitary materials that require such characteristics as water absorbability, diffusing capacity, bulkiness, anti-see-through property, and flexibility, thus ensuring an extremely large industrial value. The sanitary material products referred to herein include sanitary materials, such as water absorbable sheet material in sanitary napkins, and diapers, as described above.

### Examples

The nonwoven fabric for sanitary materials for the water absorption sheet for sanitary napkins according to the present invention is described in more detail below with reference to Examples, although the present invention is not limited only to these Examples. The methods described below were used to measure the physical properties adopted in the Examples, where the average of five measurements was used to represent each property.

### <Test method for the thickness (bulkiness) of nonwoven fabric>

Five sheets of nonwoven fabric with a targeted of 40 g/m² were stacked and the thickness of the stack was measured using a KES-G5 of Kato Tech Co., Ltd., under the standard conditions including a pressing plate descending speed of 50 sec/mm and a pressing plate area of 2 cm². The compression test run was repeated five times and the thickness under a compression load of 50 gf/cm² was calculated (average value for n = 5). A larger thickness means a higher bulkiness and permeability.
- 2.0 mm or more: ○
- less than 2.0 mm: ×

### <Test method for the water absorbability of nonwoven fabric>

A sheet of nonwoven fabric with a metsuke of 40 g/m² was put on an acrylic plate stand set obliquely with an inclination of 45°, and a water droplet of 0.1 cc was dropped, followed by measuring the distance over which the water droplet ran. A shorter distance means a higher hydrophilicity.
- 15 mm or less: ○
- 16 mm or more: ×

### <Test method for the liquid diffusing capacity of nonwoven fabric>

A 100 mm × 100 mm sheet was cut out of nonwoven fabric with a targeted of 40 g/m² and put on a petri dish with the central portion slightly lifted, and 0.1 cc of ink was dropped to the surface of the nonwoven fabric, followed by measuring the diffusion area in 10 minutes. A larger area means a higher diffusing capacity.
- 380 mm² or more: ○
- less than 380 mm²: ×

### <Test method for the flexibility of nonwoven fabric>

Measurements were taken according to the Handle-O-Meter method specified in JIS L 1913 (2010) 6.7.5. A 200 mm × 200 mm test piece was cut out of nonwoven fabric with a metsuke of 40 g/m² and set on a specimen table, and a blade adjusted to descend to a height 8 mm below the surface of the specimen table was lowered to press down the test piece while measuring the resistance force. A larger resistance force means a higher softness.
- 180 mN or more: ○
- less than 180 mN: ×

### <Test method for the anti-see-through property (opacity) of nonwoven fabric>

Measurements were taken according to the test method for opacity of nonwoven fabric specified in JIS L 1912 (1997) 6.16. For nonwoven fabric with a targeted of 40 g/m², the reflectance of nonwoven fabric specimen sheet backed by a black cylinder was measured by a photometer, and its percent ratio to the intrinsic reflectance of a stack of a sufficiently large number of specimen sheets of the same nonwoven fabric to prevent changes in reflectance was calculated. A larger ratio means a higher opacity.
- 50% or more: ○
- less than 50%: ×

### <Overall evaluation>

For overall evaluation in the above physical properties, a specimen was evaluated as ○ (acceptable) if ranked as ○ in all of the tests for nonwoven fabric thickness (bulkiness), water absorbability, liquid diffusing capacity, flexibility, and anti-see-through property (opacity), evaluated as Δ if ranked as × in any of the tests, and evaluated as × if ranked as × in all of the tests, and it was judged to be unacceptable if evaluated as Δ or ×.

### [Example 1]

Using a spinneret containing special irregular-shaped holes for flattened multilobar cross section formation, polyethylene terephthalate with a molten polymer density of 1.18 g/cc was spun under the conditions of a spinning temperature of 290°C, a discharge rate of 400 g/min, a cooling air speed of 60 m/min, a cooling temperature of 25°C, and a take-up speed of 1,200 m/min to produce an undrawn yarn. The resulting undrawn yarn was stretched 3.3 times and crimped with a frequency of 13 crimps/25 mm and a crimp rate of 14%, and a hydrophilizing agent (nonionic surface active agent containing a hydrophilic polyester component) is added to the fiber surface to 0.3 mass%, followed by drying at a temperature of 120°C. After the drying, the resulting yarn was cut to 51 mm to provide polyester fiber (with a single filament fineness of 1.7 dtex) with a flattened multilobar cross section that had a flatness of 2.1, a degree of irregularity of 2.0, a convex ratio of 0.6, and a cross-sectional shape containing 8 convex portions.

The polyester fiber thus obtained (100 mass%) with a flattened multilobar cross section was carded by an ordinary method to form a fiber web with a metsuke of 50 g/cm², and a sheet was prepared by bifacial processing under the conditions of a water jet pressure of 50 kg/cm² and a water jet speed of 1 m/min using a nozzle having a 0.1 mm diameter, 0.6 mm pitch, 834 holes, and 500 mm effective width, followed by performing heat treatment by the air-through method at a temperature of 150°C to provide nonwoven fabric with a metsuke of 40 g/m². The fiber constitution of the resulting nonwoven fabric for sanitary materials is shown in Table 1 and its evaluation results are given in Table 2.

### [Example 2]

Using a spinneret containing special irregular-shaped holes for flattened multilobar cross section formation, polyethylene terephthalate with a molten polymer density of 1.18 g/cc was spun under the conditions of a spinning temperature of 285°C, a discharge rate of 400 g/min, a cooling air speed of 80 m/min, a cooling temperature of 20°C, and a take-up speed of 1,200 m/min to produce an undrawn yarn. The resulting undrawn yarn is stretched 3.3 times and crimped with a frequency of 13 crimps/25 mm and a crimp rate of 14%, and a hydrophilizing agent is added to the fiber surface to 0.3 mass%, followed by drying at a temperature of 120°C. After the drying, the resulting yarn was cut to 51 mm to provide polyester fiber (with a single filament fineness of 1.7 dtex) with a flattened multilobar cross section that had a flatness of 2.7, a degree of irregularity of 4.0, a convex ratio of 0.8, and a cross-sectional shape containing 8 convex portions.

The polyester fiber thus obtained (100 mass%) with a flattened multilobar cross section was carded by an ordinary method to form a fiber web with a metsuke of 50 g/cm², and a sheet was prepared by bifacial processing under the conditions of a water jet pressure of 50 kg/cm² and a water jet speed of 1 m/min using a nozzle having a 0.1 mm diameter, 0.6 mm pitch, 834 holes, and 500 mm effective width, followed by performing heat treatment by the air-through method at a temperature of 150°C to provide nonwoven fabric with a targeted of 40 g/m². The fiber constitution of the resulting nonwoven fabric for sanitary materials is shown in Table 1 and its evaluation results are given in Table 2.

### [Example 3]

Using a spinneret containing special irregular-shaped holes for flattened multilobar cross section formation, polyethylene terephthalate with a molten polymer density of 1.18 g/cc was spun under the conditions of a spinning temperature of 290°C, a discharge rate of 400 g/min, a cooling air speed of 60 m/min, a cooling temperature of 25°C, and a take-up speed of 1,200 m/min to produce an undrawn yarn. The resulting undrawn yarn was stretched 3.3 times and crimped with a frequency of 13 crimps/25 mm and a crimp rate of 14%, and a hydrophilizing agent is added to the fiber surface to 0.3 mass%, followed by drying at a temperature of 120°C. After the drying, the resulting yarn was cut to 51 mm to provide polyester fiber (with a single filament fineness of 1.7 dtex) with a flattened multilobar cross section that had a flatness of 2.1, a degree of irregularity of 2.0, a convex ratio of 0.6, and a cross-sectional shape containing 8 convex portions.

A uniform mixture of 90 mass% of the polyester fiber thus obtained with a flattened multilobar cross section and 10 mass% of polyethylene fiber (with a monofilament fineness of 1.7 dtex and a fiber length of 51 mm) was carded by an ordinary method to form a fiber web with a metsuke of 50 g/cm², and a sheet was prepared by bifacial processing under the conditions of a water jet pressure of 50 kg/cm² and a water jet speed of 1 m/min using a nozzle having a 0.1 mm diameter, 0.6 mm pitch, 834 holes, and 500 mm effective width, followed by performing heat treatment by the air-through method at a temperature of 150°C to provide nonwoven fabric with a targeted of 40 g/m². The fiber constitution of the resulting nonwoven fabric for sanitary materials is shown in Table 1 and its evaluation results are given in Table 2.

### [Example 4]

Using a spinneret containing special irregular-shaped holes for flattened multilobar cross section formation, polyethylene terephthalate with a molten polymer density of 1.18 g/cc was spun under the conditions of a spinning temperature of 285°C, a discharge rate of 400 g/min, a cooling air speed of 80 m/min, a cooling temperature of 20°C, and a take-up speed of 1,200 m/min to produce an undrawn yarn. The resulting undrawn yarn is stretched 3.3 times and crimped with a frequency of 13 crimps/25 mm and a crimp rate of 14%, and a hydrophilizing agent is added to the fiber surface to 0.3 mass%, followed by drying at a temperature of 120°C. After the drying, the resulting yarn was cut to 51 mm to provide polyester fiber (with a single filament fineness of 1.7 dtex) with a flattened multilobar cross section that had a flatness of 2.7, a degree of irregularity of 4.0, a convex ratio of 0.8, and a cross-sectional shape containing 8 convex portions.

A uniform mixture of 90 mass% of the polyester fiber thus obtained with a flattened multilobar cross section and 10 mass% of polyethylene fiber (with a monofilament fineness of 1.7 dtex and a fiber length of 51 mm) was carded by an ordinary method to form a fiber web with a metsuke of 50 g/cm², and a sheet was prepared by bifacial processing under the conditions of a water jet pressure of 50 kg/cm² and a water jet speed of 1 m/min using a nozzle having a 0.1 mm diameter, 0.6 mm pitch, 834 holes, and 500 mm effective width, followed by performing heat treatment by the air-through method at a temperature of 150°C to provide nonwoven fabric with atargeted of 40 g/m². The fiber constitution of the resulting nonwoven fabric for sanitary materials is shown in Table 1 and its evaluation results are given in Table 2.

### [Example 5]

Using a spinneret containing special irregular-shaped holes for flattened multilobar cross section formation, polyethylene terephthalate material with a molten polymer density of 1.18 g/cc was spun under the conditions of a spinning temperature of 290°C, a discharge rate of 400 g/min, a cooling air speed of 60 m/min, a cooling temperature of 25°C, and a take-up speed of 1,200 m/min to produce an undrawn yarn. The resulting undrawn yarn was stretched 3.3 times and crimped with a frequency of 13 crimps/25 mm and a crimp rate of 14%, and a hydrophilizing agent is added to the fiber surface to 0.3 mass%, followed by drying at a temperature of 120°C. After the drying, the resulting yarn was cut to 51 mm to provide polyester fiber (with a single filament fineness of 1.7 dtex) with a flattened multilobar cross section that had a flatness of 2.1, a degree of irregularity of 2.0, a convex ratio of 0.6, and a cross-sectional shape containing 8 convex portions.

A uniform mixture of 70 mass% of the polyester fiber thus obtained with a flattened multilobar cross section, 20 mass% of rayon fiber (with a monofilament fineness of 1.7 dtex and a fiber length of 51 mm), and 10 mass% of polyethylene fiber (with a monofilament fineness of 1.7 dtex and a fiber length of 51 mm) was carded by an ordinary method to form a fiber web with a metsuke of 50 g/cm², and a sheet was prepared by bifacial processing under the conditions of a water jet pressure of 50 kg/cm² and a water jet speed of 1 m/min using a nozzle having a 0.1 mm diameter, 0.6 mm pitch, 834 holes, and 500 mm effective width, followed by performing heat treatment by the air-through method at a temperature of 150°C to provide nonwoven fabric with a targeted of 40 g/m². The fiber constitution of the resulting nonwoven fabric for sanitary materials is shown in Table 1 and its evaluation results are given in Table 2.

### [Example 6]

Using a spinneret containing special irregular-shaped holes for flattened multilobar cross section formation, polyethylene terephthalate with a molten polymer density of 1.18 g/cc was spun under the conditions of a spinning temperature of 285°C, a discharge rate of 400 g/min, a cooling air speed of 80 m/min, a cooling temperature of 20°C, and a take-up speed of 1,200 m/min to produce an undrawn yarn. The resulting undrawn yarn is stretched 3.3 times and crimped with a frequency of 13 crimps/25 mm and a crimp rate of 14%, and a hydrophilizing agent is added to the fiber surface to 0.3 mass%, followed by drying at a temperature of 120°C. After the drying, the resulting yarn was cut to 51 mm to provide polyester fiber (with a single filament fineness of 1.7 dtex) with a flattened multilobar cross section that had a flatness of 2.7, a degree of irregularity of 4.0, a convex ratio of 0.8, and a cross-sectional shape containing 8 convex portions.

A uniform mixture of 70 mass% of the polyester fiber thus obtained with a flattened multilobar cross section, 20 mass% of rayon fiber (with a monofilament fineness of 1.7 dtex and a fiber length of 51 mm), and 10 mass% of polyethylene fiber (with a monofilament fineness of 1.7 dtex and a fiber length of 51 mm) was carded by an ordinary method to form a fiber web with a metsuke of 50 g/cm², and a sheet was prepared by bifacial processing under the conditions of a water jet pressure of 50 kg/cm² and a water jet speed of 1 m/min using a nozzle having a 0.1 mm diameter, 0.6 mm pitch, 834 holes, and 500 mm effective width, followed by performing heat treatment by the air-through method at a temperature of 150°C to provide nonwoven fabric with a targeted of 40 g/m². The fiber constitution of the resulting nonwoven fabric for sanitary materials is shown in Table 1 and its evaluation results are given in Table 2.

**[Table 1]**

| | Example 1 | Example 2 | Example 3 | Example 4 | Example 5 | Example 6 |
|---|---|---|---|---|---|---|
| Polyester fiber with flattened multilobar cross section (flatness 2.1) | 100 | | 90 | | 70 | |
| Polyester fiber with flattened multilobar cross section (flatness 2.7) | | 100 | | 90 | | 70 |
| Polyester fiber with flattened cross section | | | | | | |
| Y-shaped (trilobate) cross section polyester fiber | | | | | | |
| Cruciform cross section polyester fiber | | | | | | |
| H-shaped cross section polyester fiber | | | | | | |
| Circular cross section polyester fiber | | | | | | |
| Rayon fiber | | | | | 20 | 20 |
| Polyethylene fiber | | | 10 | 10 | 10 | 10 |

**[Table 2]**

| | Example 1 | Example 2 | Example 3 | Example 4 | Example 5 | Example 6 |
|---|---|---|---|---|---|---|
| Thickness of nonwoven fabric (mm) | ○ (2.3) | ○ (2.4) | ○ (2.2) | ○ (2.3) | ○ (2.1) | ○ (2.3) |
| Water absorbability of nonwoven fabric (mm) | ○ (12) | ○ (11) | ○ (13) | ○ (11) | ○ (11) | ○ (10) |
| Liquid diffusing capacity of nonwoven fabric (mm²) | ○ (410) | ○ (395) | ○ (400) | ○ (390) | ○ (420) | ○ (405) |
| Flexibility of nonwoven fabric (mN) | ○ (198) | ○ (203) | ○ (190) | ○ (192) | ○ (192) | ○ (188) |
| Anti-see-through of nonwoven fabric: Opacity (%) | ○ (55) | ○ (56) | ○ (56) | ○ (58) | ○ (56) | ○ (59) |
| Overall evaluation | ○ | ○ | ○ | ○ | ○ | ○ |

### [Comparative example 1]

A uniform mixture of 70 mass% of polyester fiber with a flattened cross section having a flatness of 2.5 and a degree of irregularity of 0 (with a single filament fineness of 1.7 dtex and a fiber length of 51 mm), 20 mass% of rayon fiber (with a single filament fineness of 1.7 dtex and a fiber length of 51 mm), and 10 mass% of polyethylene fiber (with a single filament fineness of 1.7 dtex and a fiber length of 51 mm) was carded by an ordinary method to form a fiber web with a targeted of 50 g/cm², and a sheet was prepared by bifacial processing under the conditions of a water jet pressure of 50 kg/cm² and a water jet speed of 1 m/min using a nozzle having a 0.1 mm diameter, 0.6 mm pitch, 834 holes, and 500 mm effective width, followed by performing heat treatment by the air-through method at a temperature of 150°C to provide nonwoven fabric with a targeted of 40 g/m². The fiber constitution of the resulting nonwoven fabric is shown in Table 3 and its evaluation results are given in Table 4.

### [Comparative example 2]

A uniform mixture of 70 mass% of polyester fiber with a Y-shaped cross section having a flatness of 1.0, a degree of irregularity of 1.0, and 3 convex portions (with a single filament fineness of 1.7 dtex and a fiber length of 51 mm), 20 mass% of rayon fiber (with a single filament fineness of 1.7 dtex and a fiber length of 51 mm), and 10 mass% of polyethylene fiber (with a single filament fineness of 1.7 dtex and a fiber length of 51 mm) was carded by an ordinary method to form a fiber web with a targeted of 50 g/cm², and a sheet was prepared by bifacial processing under the conditions of a water jet pressure of 50 kg/cm² and a water jet speed of 1 m/min using a nozzle having a 0.1 mm diameter, 0.6 mm pitch, 834 holes, and 500 mm effective width, followed by performing heat treatment by the air-through method at a temperature of 150°C to provide nonwoven fabric with a targeted of 40 g/m². The fiber constitution of the resulting nonwoven fabric is shown in Table 3 and its evaluation results are given in Table 4.

### [Comparative example 3]

A uniform mixture of 70 mass% of polyester fiber with a cruciform cross section having a flatness of 1.0, a degree of irregularity of 3.0, and 4 convex portions (with a single filament fineness of 1.7 dtex and a fiber length of 51 mm), 20 mass% of rayon fiber (with a single filament fineness of 1.7 dtex and a fiber length of 51 mm), and 10 mass% of polyethylene fiber (with a single filament fineness of 1.7 dtex and a fiber length of 51 mm) was carded by an ordinary method to form a fiber web with a targeted of 50 g/cm², and a sheet was prepared by bifacial processing under the conditions of a water jet pressure of 50 kg/cm² and a water jet speed of 1 m/min using a nozzle having a 0.1 mm diameter, 0.6 mm pitch, 834 holes, and 500 mm effective width, followed by performing heat treatment by the air-through method at a temperature of 150°C to provide nonwoven fabric with a targeted of 40 g/m². The fiber constitution of the resulting nonwoven fabric is shown in Table 3 and its evaluation results are given in Table 4.

### [Comparative example 4]

A uniform mixture of 70 mass% of polyester fiber with a H-shaped cross section having a flatness of 1.0, a degree of irregularity of 2.3, and 4 convex portions (with a single filament fineness of 1.7 dtex and a fiber length of 51 mm), 20 mass% of rayon fiber (with a single filament fineness of 1.7 dtex and a fiber length of 51 mm), and 10 mass% of polyethylene fiber (with a single filament fineness of 1.7 dtex and a fiber length of 51 mm) was carded by an ordinary method to form a fiber web with a targeted of 50 g/cm², and a sheet was prepared by bifacial processing under the conditions of a water jet pressure of 50 kg/cm² and a water jet speed of 1 m/min using a nozzle having a 0.1 mm diameter, 0.6 mm pitch, 834 holes, and 500 mm effective width, followed by performing heat treatment by the air-through method at a temperature of 150°C to provide nonwoven fabric with a targeted of 40 g/m². The fiber constitution of the resulting nonwoven fabric is shown in Table 3 and its evaluation results are given in Table 4.

### [Comparative example 5]

A uniform mixture of 70 mass% of polyester fiber with a circular cross section having a flatness of 1.0 (with a single filament fineness of 1.7 dtex and a fiber length of 51 mm), 20 mass% of rayon fiber (with a single filament fineness of 1.7 dtex and a fiber length of 51 mm), and 10 mass% of polyethylene fiber (with a single filament fineness of 1.7 dtex and a fiber length of 51 mm) was carded by an ordinary method to form a fiber web with a targeted of 50 g/cm², and a sheet was prepared by bifacial processing under the conditions of a water jet pressure of 50 kg/cm² and a water jet speed of 1 m/min using a nozzle having a 0.1 mm diameter, 0.6 mm pitch, 834 holes, and 500 mm effective width, followed by performing heat treatment by the air-through method at a temperature of 150°C to provide nonwoven fabric with a targeted of 40 g/m². The fiber constitution of the resulting nonwoven fabric is shown in Table 3 and its evaluation results are given in Table 4.

**[Table 3]**

| | Comparative example 1 | Comparative example 2 | Comparative example 3 | Comparative example 4 | Comparative example 5 |
|---|---|---|---|---|---|
| Polyester fiber with flattened multilobar cross section (flatness 2.1) | | | | | |
| Polyester fiber with flattened multilobar cross section (flatness 2.7) | | | | | |
| Polyester fiber with flattened cross section | 70 | | | | |
| Y-shaped (trilobate) cross section polyester fiber | | 70 | | | |
| Cruciform cross section polyester fiber | | | 70 | | |
| H-shaped cross section polyester fiber | | | | 70 | |
| Circular cross section polyester fiber | | | | | 70 |
| Rayon fiber | 20 | 20 | 20 | 20 | 20 |
| Polyethylene fiber | 10 | 10 | 10 | 10 | 10 |

**[Table 4]**

| | Comparative example 1 | Comparative example 2 | Comparative example 3 | Comparative example 4 | Comparative example 5 |
|---|---|---|---|---|---|
| Thickness of nonwoven fabric (mm) | × (1.8) | × (1.7) | × (1.9) | × (1.9) | × (1.5) |
| Water absorbability of nonwoven fabric (mm) | ○ (13) | ○ (13) | ○ (12) | ○ (12) | × (16) |
| Liquid diffusing capacity of nonwoven fabric (mm²) | × (160) | × (230) | × (275) | × (175) | × (120) |
| Flexibility of nonwoven fabric (mN) | ○ (194) | × (165) | × (162) | ○ (180) | × (156) |
| Anti-see-through of nonwoven fabric: Opacity (%) | × (47) | ○ (51) | ○ (53) | ○ (53) | × (42) |
| Overall evaluation | Δ | Δ | Δ | Δ | × |

As seen from Examples 1 to 6, nonwoven fabrics of polyester based fiber having a fiber constitution characterized by a flattened cross section having 8 convex portions along the circumference of the cross section were so high in water absorbability and diffusing capacity that they were able to absorb liquids such as water and chemicals instantly, and the nonwoven fabrics were so high in opacity that the absorbed liquid was not significantly visible, and also so high in bulkiness and texture softness that they had a good feel to the skin, accordingly giving high-level evaluation results in all test items. Thus, they were found to be suitable for nonwoven fabrics for sanitary materials.

Compared to this, although having high bulkiness and a soft texture, nonwoven fabrics of polyester based fiber with a flattened cross section having a low degree of irregularity had a smaller diffusion area when absorbing and water suffered from wetback, i.e., returning of the liquid back to the skin through the surface material, to cause a strong discomfort, as seen from Comparative example 1.

Furthermore, as seen from Comparative examples 2 to 5, nonwoven fabrics of polyester based fiber having other low-flatness, irregularcross-sectional shapes were not only low in liquid diffusing capacity, but also poor in fabric softness and bulkiness, resulting in stiff texture and a considerably uncomfortable feel to the skin.

### Explanation of Numerals

A: The maximum length across the fiber cross section
B: The maximum width of the fiber cross section
C: The length of the line connecting the apexes of the adjacent convex portions in the largest irregular shape part of the fiber cross section
D: The length of the perpendicular line from the line C connecting two convex portions to the bottom of the concave portion
E: The length of the second largest width next to the largest width B in the cross section

## Claims

1. Water absorption sheet for sanitary napkins comprising a nonwoven fabric for sanitary materials comprising polyester based fiber with a flattened multilobar cross section containing 6 or more convex portions along the cross-sectional circumference, the surface of the nonwoven fabric for sanitary materials being covered with olefin based nonwoven fabric, the polyester based fiber with a flattened multilobar cross section meeting the following requirements (1) to (3) simultaneously:
• flatness (A/B) = 2.0 to 3.0, (1)
• degree of irregularity (C/D) = 1.0 to 5.0, and (2)
• convex ratio (E/B) = 0.6 to 0.9, (3)
wherein
A: A denotes the maximum length across the fiber cross section,
B: B denotes the maximum width of the fiber cross section,
C: C denotes the length of the line connecting the apexes of the adjacent convex portions in the largest irregular shape part of the fiber cross section,
D: D denotes the length of the line that extends perpendicularly from the line C connecting two convex portions to the bottom of the concave portion, and
E: E denotes the length of the second longest width next to the maximum width B of the cross section.

2. Water absorption sheet for sanitary napkins comprising a nonwoven fabric for sanitary materials as set forth in claim 1, wherein the polyester based fiber with a flattened multilobar cross section has a single filament fineness of 2.0 dtex or less.

3. Water absorption sheet for sanitary napkins comprising a nonwoven fabric for sanitary materials as set forth in either claim 1 or 2, wherein the polyester based fiber with a flattened multilobar cross section has a fiber length in the range of 3 mm to 64 mm.

4. Water absorption sheet for sanitary napkins comprising a nonwoven fabric for sanitary materials as set forth in any one of claims 1 to 3, wherein the polyester based fiber with a flattened multilobar cross section is joined by heat-weldable fiber.

## Patentansprüche

1. Wasserabsorbierende Lage für Hygienebinden, die einen Vliesstoff für Hygienematerialien aufweist, der Fasern auf Polyesterbasis mit einem abgeflachten mehrlappigen Querschnitt aufweist, der 6 oder mehr konvexe Abschnitte entlang des Querschnittsumfangs enthält, wobei die Oberfläche des Vliesstoffs für Hygienematerialien mit Vliesstoff auf Olefinbasis bedeckt ist, wobei die Faser auf Polyesterbasis mit einem abgeflachten mehrlappigen Querschnitt gleichzeitig die folgenden Anforderungen (1) bis (3) erfüllt:
• Ebenheit (A/B) = 2,0 bis 3,0, (1)
• Grad der Unregelmäßigkeit (C/D) = 1,0 bis 5,0, und (2)
• Konvexes Verhältnis (E/B) = 0,6 bis 0,9, (3)
wobei
A: A die maximale Länge über den Faserquerschnitt bezeichnet,
B: B die maximale Breite des Faserquerschnitts bezeichnet,
C: C die Länge der Linie bezeichnet, die die Scheitelpunkte der benachbarten konvexen Abschnitte in dem Teil mit der größten unregelmäßigen Form des Faserquerschnitts verbindet,
D: D die Länge der Linie bezeichnet, die sich senkrecht von der Linie C, die zwei konvexe Abschnitte verbindet, zum Boden des konkaven Abschnitts erstreckt, und
E: E die Länge der zweitlängsten Breite neben der maximalen Breite B des Querschnitts bezeichnet.

2. Wasserabsorbierende Lage für Hygienebinden, die einen Vliesstoff für Hygienematerialien aufweist, nach Anspruch 1, wobei die Faser auf Polyesterbasis mit einem abgeflachten mehrlappigen Querschnitt eine Einzelfadenfeinheit von 2,0 dtex oder weniger hat.

3. Wasserabsorbierende Lage für Hygienebinden, die einen Vliesstoff für Hygienematerialien aufweist, nach Anspruch 1 oder 2, wobei die Faser auf Polyesterbasis mit einem abgeflachten mehrlappigen Querschnitt eine Faserlänge im Bereich von 3 mm bis 64 mm hat.

4. Wasserabsorbierende Lage für Hygienebinden, die einen Vliesstoff für Hygienematerialien aufweist, nach einem der Ansprüche 1 bis 3, wobei die Faser auf Polyesterbasis mit einem abgeflachten mehrlappigen Querschnitt durch heißschweißbare Fasern verbunden ist.

## Revendications

1. Feuille d'absorption d'eau pour serviettes hygiéniques comprenant un tissu non tissé pour articles hygiéniques comprenant une fibre à base de polyester ayant une section transversale multilobée aplatie contenant 6 parties convexes ou plus le long de la circonférence de section transversale, la surface du tissu non tissé pour articles hygiéniques étant recouverte d'un tissu non tissé à base d'oléfine, la fibre à base de polyester ayant une section transversale multilobée aplatie répondant aux exigences (1) à (3) suivantes simultanément :
• planéité (A/B) = 2,0 à 3,0, (1)
• degré d'irrégularité (C/D) = 1,0 à 5,0 et (2)
• rapport de convexité (E/B) = 0,6 à 0,9, (3)
où
A : A désigne la longueur maximale sur toute la section transversale de fibre,
B : B désigne la largeur maximale de la section transversale de fibre,
C : C désigne la longueur de la ligne reliant les sommets des parties convexes adjacentes dans la plus grande partie de forme irrégulière de la section transversale de fibre,
D : D désigne la longueur de la ligne qui s'étend perpendiculairement de la ligne C reliant deux parties convexes au fond de la partie concave, et
E : E désigne la longueur de la deuxième plus longue largeur à côté de la largeur maximale B de la section transversale.

2. Feuille d'absorption d'eau pour serviettes hygiéniques comprenant un tissu non tissé pour articles hygiéniques selon la revendication 1, dans laquelle la fibre à base de polyester ayant une section transversale multilobée aplatie a une finesse de filament individuel inférieure ou égale à 2,0 dtex.

3. Feuille d'absorption d'eau pour serviettes hygiéniques comprenant un tissu non tissé pour articles hygiéniques selon la revendication 1 ou 2, dans laquelle la fibre à base de polyester ayant une section transversale multilobée aplatie a une longueur de fibre se trouvant dans la plage allant de 3 mm à 64 mm.

4. Feuille d'absorption d'eau pour serviettes hygiéniques comprenant un tissu non tissé pour articles hygiéniques selon l'une quelconque des revendications 1 à 3, dans laquelle la fibre à base de polyester ayant une section transversale multilobée aplatie est liée par une fibre thermosoudable.
